# EUROPEAN PATENT SPECIFICATION

(11) **EP 3 140 290 B1**
(45) Date of publication and mention of the grant of the patent: **06.04.2022**
(21) Application number: 15724189.4
(22) Date of filing: 07.05.2015
(51) Int. Cl.: C07D 239/94, G01N 23/20, A61K 31/517, A61P 35/00

(54) **POLYMORPH PURITY, MONITORING AND ASSOCIATED COMPOSITIONS**
POLYMORPH REINEN, ÜBERWACHUNG UND ZUGEHÖRIGE ZUSAMMENSETZUNGEN
PURETÉ POLYMORPHE, SURVEILLANCE ET COMPOSITIONS ASSOCIÉES

(30) Priority: 07.05.2014 GB 201408094; 29.05.2014 GB 201409560
(43) Date of publication of application: 15.03.2017
(73) Proprietor: Remedica Ltd, Limassol 3508 (CY)
(72) Inventor: PATTIHIS, Charalambos, 3508 Limassol (CY); NORDMANN, Antje, 3508 Limassol (CY)
(74) Representative: Isarpatent
(86) International application number: PCT/EP2015/060129
(87) International publication number: WO 2015/169932

(56) References cited:
- WO-A1-2013/156835
- WO-A2-2009/024989
- WO-A2-2011/058525
- WO-A2-2014/136126
- 3 KW GENERATOR WITHOUT INTERNAL COOLING UNIT XP055740747

## Description

The present invention is concerned with a method of monitoring polymorph purity, in relation to erlotinib, and a process for preparing a pharmaceutical composition comprising erlotinib hydrochloride Form A.

Erlotinib, N-(3-ethynylphenyl)-6,7-bis(2-methoxyethoxy)-4-quinazolinamine, has the following structural Formula

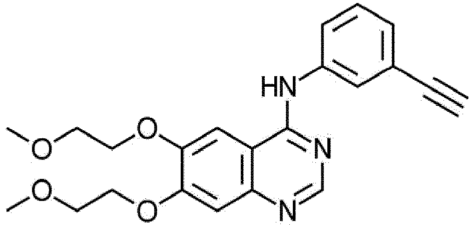

WO 01/34574 discloses erlotinib hydrochloride Forms A and B. WO 01/34574 teaches that Form A is thermodynamically less stable than Form B. In particular, page 16 of WO 01/34574 discloses that polymorph B of erlotinib hydrochloride was found to be the thermodynamically most stable and desirable Form. On this basis, WO 01/34574 aimed to provide Form B in the substantially pure polymorphic Form, and also pharmaceutical compositions of the substantially pure Form B, particularly as a tablet and a method of the selective production of the compound. Thus although Form A is disclosed in WO 01/34575, the main aim of this document was to disclose pure Form B and compositions including the same.

Also according to WO 01/34574, the disclosure of the original compound patent for erlotinib, namely US 5747498, actually disclosed the provision of a mixture of the polymorphs Form A and B. Still further according to WO 01/34574, because of the hydrochloride polymorph reduced stability, in particular due to the thermodynamically less stable Form A component, the hydrochloride salt was not more preferred for tablet formulation than the mesylate salt forms.

Specifically according to WO 01/34574, therefore, there was disclosed substantially homogeneous erlotinib hydrochloride Form B that exhibits an X-ray powder diffraction pattern having characteristic peaks expressed in degrees 2-theta at approximately 6.26, 12.48, 13.39, 16.96, 20.20, 21.10, 22.98, 24.46, 25.14 and 26.91. Form B was also characterized in WO 01/34574 by the X-ray powder diffraction pattern shown in Figure 3 of WO 01/34574.

WO 01/34574 also disclosed erlotinib hydrochloride Form B that exhibits an X-ray powder diffraction pattern having characteristic peaks expressed in degrees 2-theta at approximately 6.26, 12.48, 13.39, 16.96, 20.20, 21.10, 22.98, 24.46, 25.14 and, 26.91, which is substantially free of the erlotinib hydrochloride polymorph designated the A polymorph.

Erlotinib hydrochloride Form B is in particular characterized by the above peak as disclosed in WO 01/34574 at 6.26 degrees 2-theta, although it can be appreciated that there is some measurement tolerance with different X-ray powder diffraction apparatus and conditions.

Also according to WO 01/34574, there is disclosed a pharmaceutical composition for the treatment of a hyperproliferative disorder in a mammal which substantially comprises a therapeutically effective amount of the polymorph Form B and a pharmaceutically acceptable carrier. The pharmaceutical composition may be adapted for oral administration. It may be in the form of a tablet.

Still further according to WO 01/34574, there is disclosed a composition consisting essentially of erlotinib hydrochloride polymorph Form A, which is characterized by an X-ray powder diffraction pattern shown in Figure 1 of WO 01/34574. Form A is further characterized in WO 01/34574 by the peaks shown in Table 1 or Table 2 of WO 01/34574 as below.

| d(A) | l(rel) | d(A) | l(rel) | d(A) | l(rel) | d(A) | l(rel) | d(A) | l(rel) |
|---|---|---|---|---|---|---|---|---|---|
| 15.82794 | 100.0 | 6.63179 | 1.7 | 4.55453 | 4.8 | 3.61674 | 8.2 | 2.91238 | 3.5 |
| 14.32371 | 3.9 | 5.84901 | 2.1 | 4.19685 | 4.7 | 3.50393 | 9.3 | 2.73148 | 3.7 |
| 11.74376 | 1.5 | 5.69971 | 2.3 | 4.16411 | 4.4 | 3.40200 | 6.0 | 2.60193 | 1.8 |
| 11.03408 | 1.2 | 5.46922 | 2.4 | 3.97273 | 4.7 | 3.35174 | 5.3 | 2.48243 | 1.3 |
| 10.16026 | 1.4 | 5.21396 | 3.6 | 3.91344 | 12.4 | 3.29005 | 4.2 | 2.40227 | 2.2 |
| 8.98039 | 13.1 | 4.80569 | 3.5 | 3.78223 | 24.2 | 3.05178 | 7.1 | 2.31297 | 1.7 |
| 7.85825 | 7.8 | 4.70077 | 12.2 | 3.67845 | 8.8 | 2.99750 | 3.0 | | |

| 2-Theta | l(rel) | 2-Theta | l(rel) | 2-Theta | l(rel) | 2-Theta | l(rel) | 2-Theta | l(rel) |
|---|---|---|---|---|---|---|---|---|---|
| 5.579 | 100.00 | 13.340 | 1.7 | 19.517 | 4.8 | 24.594 | 8.2 | 30.673 | 3.5 |
| 6.165 | 3.9 | 15.135 | 2.1 | 21.152 | 4.7 | 25.398 | 9.3 | 32.759 | 3.7 |
| 7.522 | 1.5 | 15.534 | 2.3 | 21.320 | 4.4 | 26.173 | 6.0 | 34.440 | 1.8 |
| 8.006 | 1.2 | 16.193 | 2.4 | 22.360 | 4.7 | 26.572 | 5.3 | 36.154 | 1.3 |
| 8.696 | 1.4 | 16.991 | 3.6 | 22.703 | 12.4 | 27.080 | 4.2 | 37.404 | 2.2 |
| 9.841 | 13.1 | 18.447 | 3.5 | 23.502 | 24.2 | 29.240 | 7.1 | 38.905 | 1.7 |
| 11.251 | 7.8 | 18.862 | 12.2 | 24.175 | 8.8 | 30.007 | 3.0 | | |

The time per step for the above analysis is 1.0 second.

What is particularly noticeable from the above, is the low intensity peak given at 6.165 degrees 2-theta in WO 01/34574 as being part of the 2-theta characterization allegedly attributable to Form A. This 6.165 peak is, however, indicative of Form B contamination in the Form A sample of WO 01/34574. Such contamination is not surprising given the thermodynamic instability associated with Form A as taught by WO 01/34574.

WO 01/34574 also discloses the following characterization of Form A, namely an X-ray powder diffraction pattern having characteristic peaks expressed in degrees 2-theta at approximately 5.58, 9.84, 11.25, 18.86, 22.70, 23.50, 24.18, 24.59, 25.40 and 29.24.

WO 2009/024989 reports that the erlotinib hydrochloride crystalline polymorph Form A obtained by the processes described in the art, for example by the processes described and exemplified in the US 5747498 and WO 01/34574, were contaminated with polymorph Form B. Specifically, the experimental data disclosed in WO 01/34574 showed that the polymorph Form A had a peak in an X-ray powder diffraction pattern at about 6.26 ± 0.2 degrees 2-theta, which is the characteristic peak of polymorph Form B as discussed above. This WO 2009/024989 analysis agrees with our interpretation of WO 01/34574 as provided above.

According to WO 2009/024989, therefore, there was a need in the art for a process for producing erlotinib hydrochloride crystalline polymorph Form A substantially free of polymorph Form B. Thus, there was allegedly provided by WO 2009/024989 erlotinib hydrochloride crystalline polymorph Form A substantially free of polymorph Form B, and specifically erlotinib hydrochloride crystalline polymorph Form A characterized by peaks in the powder X-ray diffraction pattern having 2-theta angle positions at about 5.75, 9.88, 11.40, 18.97, 22.84, 23.65, 24.29, 24.75, 25.56 and 29.37 ± 0.2 degrees 2-theta and by the absence of a peak at about 6.26 ± 0.2 degrees 2-theta.

Also according to WO 2009/024989, there was provided a pharmaceutical composition comprising erlotinib hydrochloride crystalline polymorph Form A characterized by peaks in the powder X-ray diffraction pattern having 2-theta angle positions at about 5.75, 9.88, 11.40, 18.97, 22.84, 23.65, 24.29, 24.75, 25.56 and 29.37 ± 0.2 degrees and by the absence of a peak at about 6.26 ± 0.2 degrees 2-theta.

WO 2009/024989 further describes that the term "erlotinib hydrochloride crystalline polymorph Form A substantially free of polymorph Form B" refers to the erlotinib hydrochloride polymorph Form A containing less than about 10% crystalline polymorph Form B of erlotinib hydrochloride, preferably less than 5% crystalline polymorph Form B of erlotinib hydrochloride, more preferably less than 1% crystalline polymorph Form B of erlotinib hydrochloride, and still more preferably essentially free of crystalline polymorph Form B of erlotinib hydrochloride. "Essentially free of crystalline polymorph Form B of erlotinib hydrochloride" means that no crystalline polymorph Form B of erlotinib hydrochloride can be detected within the limits of a powder X-ray diffractometer. However, such statement in WO 2009/024989 can only be verified in practice by the authors based on the specific apparatus and associated operating conditions from WO 2009/024989.

WO 2009/024989 further describes that the X-ray powder diffraction spectrum as referenced therein was measured on a bruker axs D8 advance X-ray powder diffractometer having a copper-ka radiation. Approximately 1 gm of sample was gently flattened on a sample holder and scanned from 2 to 50 degrees two-theta, at 0.03 degrees two-theta per step and a time per step of 0.5 seconds. The sample was simply placed on the sample holder. The sample was rotated at 30 rpm at a voltage 40KV and 35 mA. However, when one looks at the specific X-ray powder diffraction analysis provided in WO 2009/024989, this can be seen to be of unacceptable sensitivity and in particular the noise level is too high for the parameters used. Thus, there is no enabling disclosure in WO 2009/024989 as to the provision of pure erlotinib hydrochloride Form A.

WO2014136126A2, WO2013156835A1, WO2011058525A2, and WO2009024989A2 describe a process for preparing erlotinib hydrochloride Form A and/or erlotinib hydrochloride Form B.

Further to the above discussion, therefore, we have now discovered that while both WO 01/34574 and WO 2009/024989 allege to provide erlotinib hydrochloride Form A, neither processes of preparation, nor X-ray powder diffraction techniques, meaningfully and reliably enable the provision of sufficiently pure erlotinib hydrochloride Form A, whereby a composition including the same as an input API would have the required stability profile on storage and / or dissolution profile. We have, therefore, now developed a process for preparing a pharmaceutical composition comprising erlotinib hydrochloride, together with one or more pharmaceutically acceptable carriers, wherein the erlotinib hydrochloride is present as Form A and which composition has acceptable storage and dissolution properties. In particular, the compositions obtained are essentially free of erlotinib hydrochloride Form B, when analysed by the X-ray powder diffraction techniques as described herein.

There is provided by the present invention a method of monitoring polymorphic purity of an erlotinib hydrochloride sample, the method comprising an X-ray powder diffraction analysis that includes a differentiation resolution and a 2-theta scan region that enables detection of the absence, or presence, of a characteristic 2-theta peak of erlotinib hydrochloride Form B in the region of 6.0 to 6.4 degrees 2-theta, which has a detection limit that enables detection of the presence of equal to, or less than, 0.2% erlotinib hydrochloride Form B in a test sample, wherein the X-ray powder diffraction analysis is operated at a current of 40 mA, a voltage of 40 kV, a step size of 0.02 degrees 2-theta and a time per step of 4.0 seconds, and with a divergence slit of 0.6 mm.

It is further preferred that the above X-ray powder diffraction analysis includes a differentiation resolution between adjacent 2-theta peaks of not more than about 0.1 degrees 2- theta. The above X-ray powder diffraction analysis has a detection limit that enables detection of the presence of equal to, or less than, 0.2% erlotinib hydrochloride Form B in a test sample.

In a preferred embodiment, the method employs a Bruker AXS D8, with a radiation source of Cu Kα (λ=1.54 Å) and a detector type LynxEye. It is operated with a current of 40 mA, a voltage of 40 kV, a step size of 0.02 degrees 2-theta and a time per step of 4.0 seconds.

The above analysis can be carried out at any stage of the development process, such as analysis of the API *per se*; a formulation mixture to be granulated; the granules themselves; and / or still further a final dosage form, such as a tablet core prior to coating and / or the resulting coated dosage form.

According to a further aspect of the present invention, this further provides a process for preparing a pharmaceutical composition comprising erlotinib hydrochloride Form A, and at least one pharmaceutically acceptable carrier, comprising:
obtaining a batch of erlotinib hydrochloride Form A;
analyzing the batch for the presence of erlotinib hydrochloride Form B as described herein;
preparing a pharmaceutical composition from the batch only if the batch is determined to have the required polymorphic purity of erlotinib hydrochloride Form A, whereby there is no detectable erlotinib hydrochloride Form B as described herein. The determination of polymorphic purity and the confirmation of the absence of Form B, is carried out by the X-ray powder diffraction analysis of the invention as described herein.

According to a still further aspect of the present invention, this further provides a process for preparing a packaged pharmaceutical composition comprising erlotinib hydrochloride Form A, comprising:
obtaining a pharmaceutical composition comprising erlotinib hydrochloride Form A;
analyzing the pharmaceutical composition for the presence of erlotinib hydrochloride Form B as described herein; and
packaging the pharmaceutical composition only if the erlotinib hydrochloride is determined to have the required polymorphic purity of erlotinib hydrochloride Form A, whereby there is no detectable erlotinib hydrochloride Form B as described herein. The determination of polymorphic purity and the confirmation of the absence of Form B, is carried out by the X-ray powder diffraction analysis of the invention as described herein.

According to a still further aspect of the present invention, this further provides a process of distributing a validated batch of a pharmaceutical composition comprising erlotinib hydrochloride Form A and at least one pharmaceutically acceptable carrier, comprising:
obtaining a batch of the pharmaceutical composition;
performing stability testing with a sample of the batch;
analyzing the sample of the batch for the presence of erlotinib hydrochloride Form B as described herein after said stability testing;
validating the batch for distribution only if the sample of the batch after stability testing is determined to have the required polymorphic purity of erlotinib hydrochloride Form A, whereby there is no detectable erlotinib hydrochloride Form B as described herein (the determination of polymorphic purity and the confirmation of the absence of Form B, is carried out by the X-ray powder diffraction analysis of the invention as described herein); and
distributing the validated batch.

The solid pharmaceutical composition, obtained by the process according to the present invention comprises erlotinib hydrochloride Form A, together with one or more pharmaceutically acceptable excipients, whereby such composition exhibits a stability profile whereby on storage at (i) a temperature of at least about 23°C, (ii) a relative humidity of at least about 55%, and (iii) for a period of at least about 6 months, no erlotinib hydrochloride Form B is detectable by X-ray powder diffraction analysis. For example, no characteristic erlotinib hydrochloride Form B 2-theta peak in the region of 6.0 to 6.4 degrees 2-theta is detectable by X-ray powder diffraction analysis. Preferably, the X-ray powder diffraction analysis is as herein described on a Bruker AXS D8 and with operating parameters also as herein described. Such stability profile is provided as a result of an input erlotinib hydrochloride API being of sufficient Form A purity, for example as determined by X-ray powder diffraction analysis as herein described.

Even more preferably, the solid pharmaceutical composition, obtained by the process according to the present invention, comprises erlotinib hydrochloride Form A, together with one or more pharmaceutically acceptable excipients, whereby such composition exhibits a stability profile whereby on storage at (i) a temperature of at least about 38°C, (ii) a relative humidity of at least about 70%, and (iii) for a period of at least about 6 months, no erlotinib hydrochloride Form B is detectable by X-ray powder diffraction analysis. For example, no characteristic erlotinib hydrochloride Form B 2-theta peak in the region of
6.0 to 6.4 degrees 2-theta is detectable by X-ray powder diffraction analysis. Preferably, the X-ray powder diffraction analysis is as herein described on a Bruker AXS D8 and with operating parameters also as herein described. Again, such stability profile is provided as a result of an input erlotinib hydrochloride API being of sufficient Form A purity, for example as determined by X-ray powder diffraction analysis as herein described.

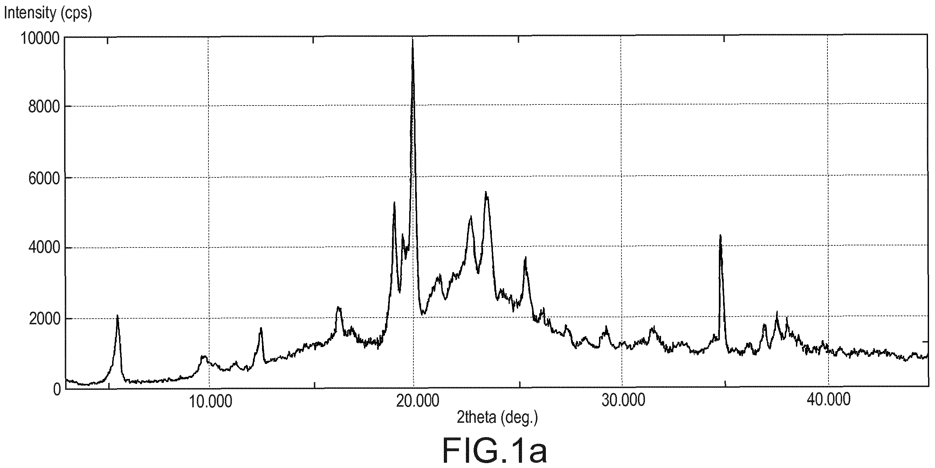

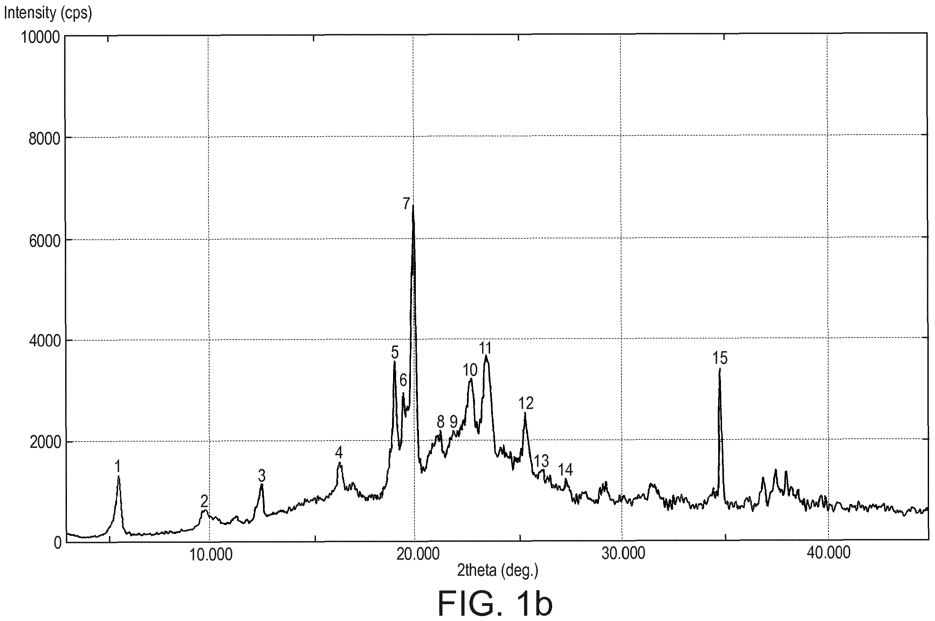

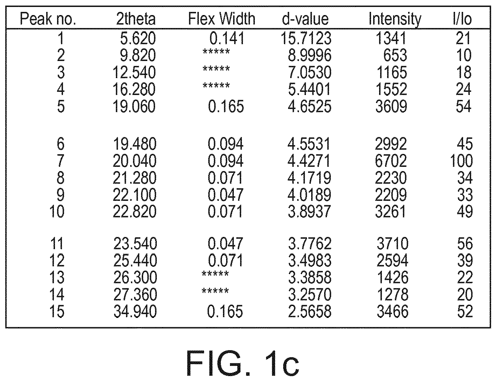

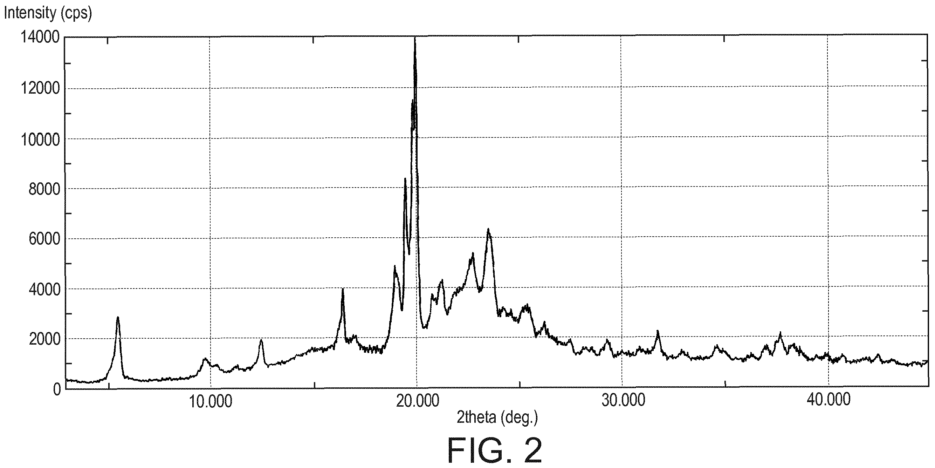

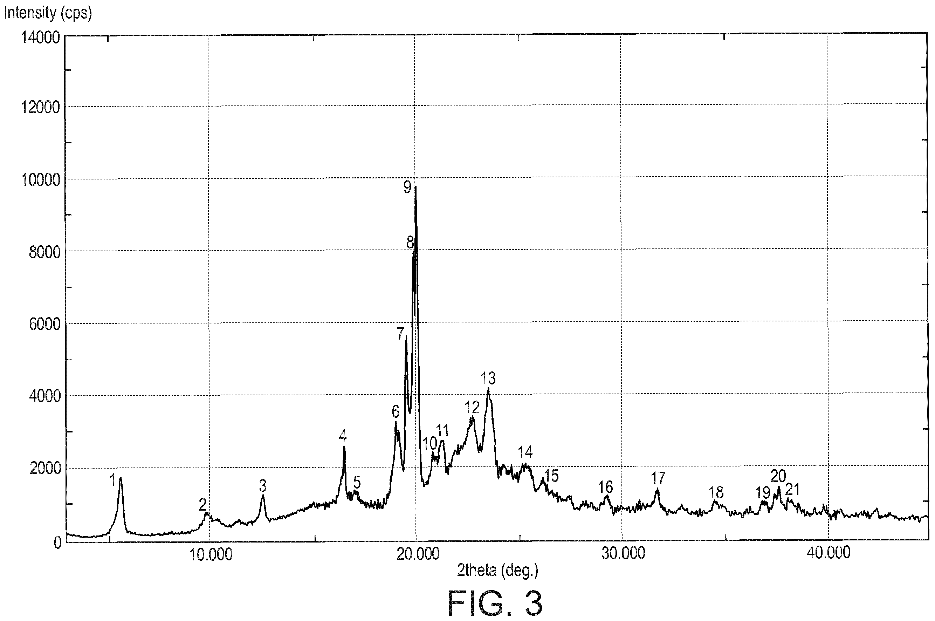

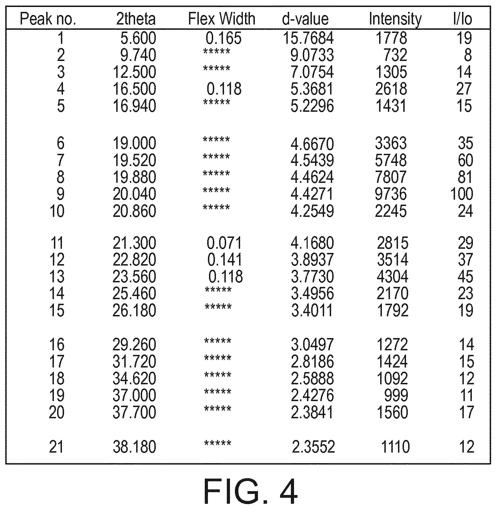

The above stability profile on storage can be supported, for example, by reference to Figures 1 to 4, that illustrate the absence of a characteristic erlotinib hydrochloride Form B 2-theta peak in the region of 6.0 to 6.4 degrees 2-theta detectable by X-ray powder diffraction analysis under accelerated storage conditions as above. The specific composition analysed in these Figures is a 150 mg compositions as set out in Example 9. The X-ray powder diffraction analysis of the tablets indicate that no transformation has occurred following 6 months of storage under accelerated conditions and pure polymorph Form A has been maintained.

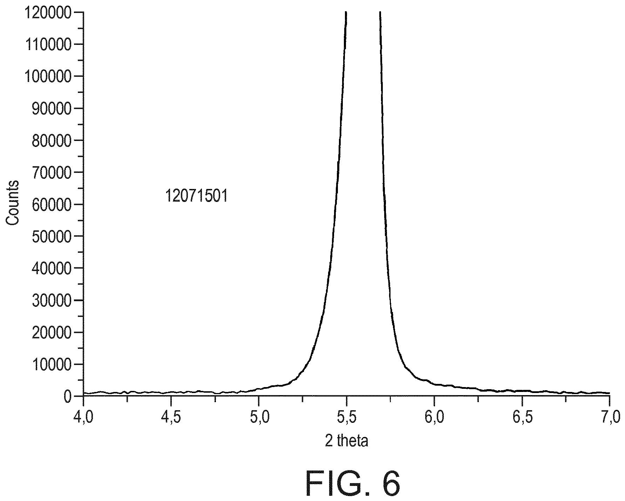

As indicated above, typically such a stability profile is provided as a result of an input erlotinib hydrochloride API being of sufficient Form A purity, for example as determined by X-ray powder diffraction analysis according to the present invention. Typically, such input API has a slow scan X-ray powder diffraction pattern showing no traces of Form B present as shown in Figure 6 and with further reference to Example 1 hereinafter.

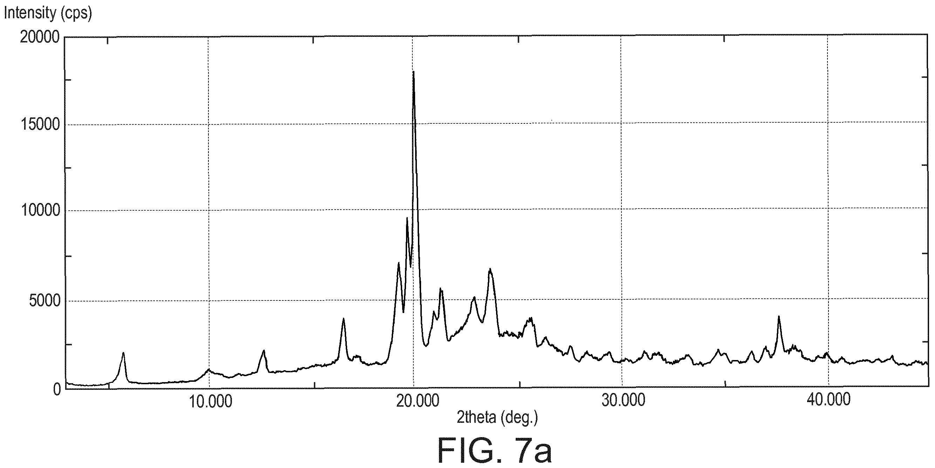

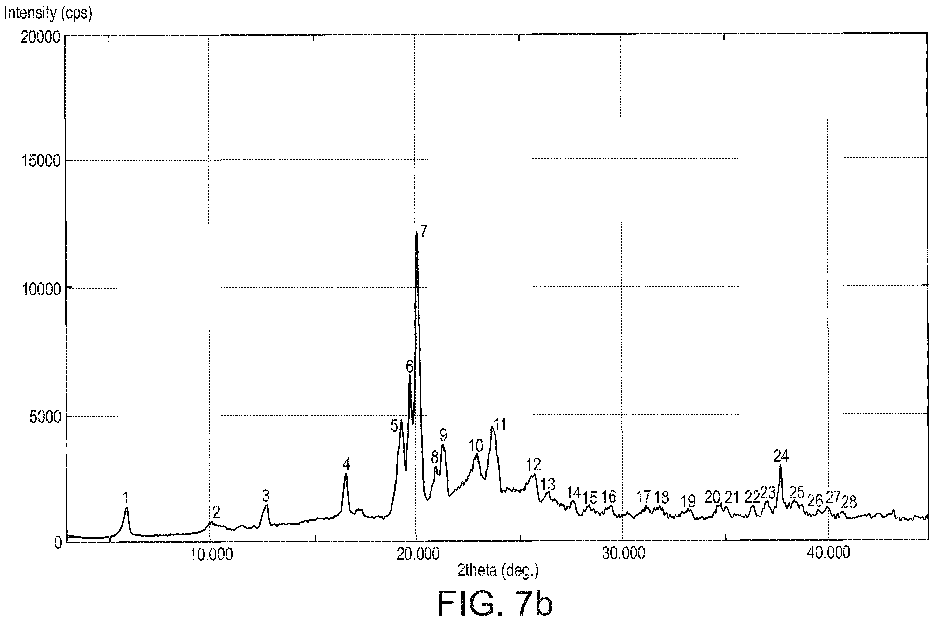

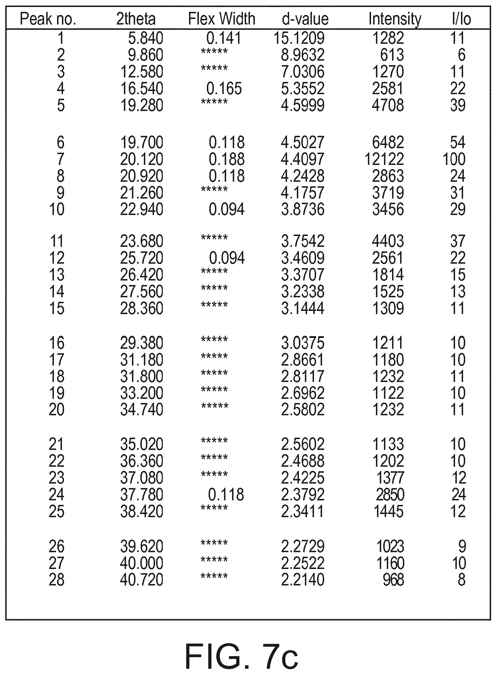

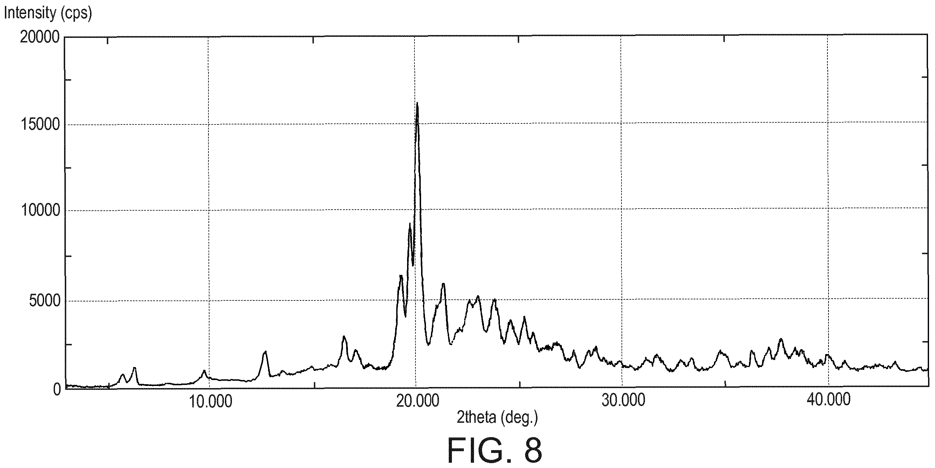

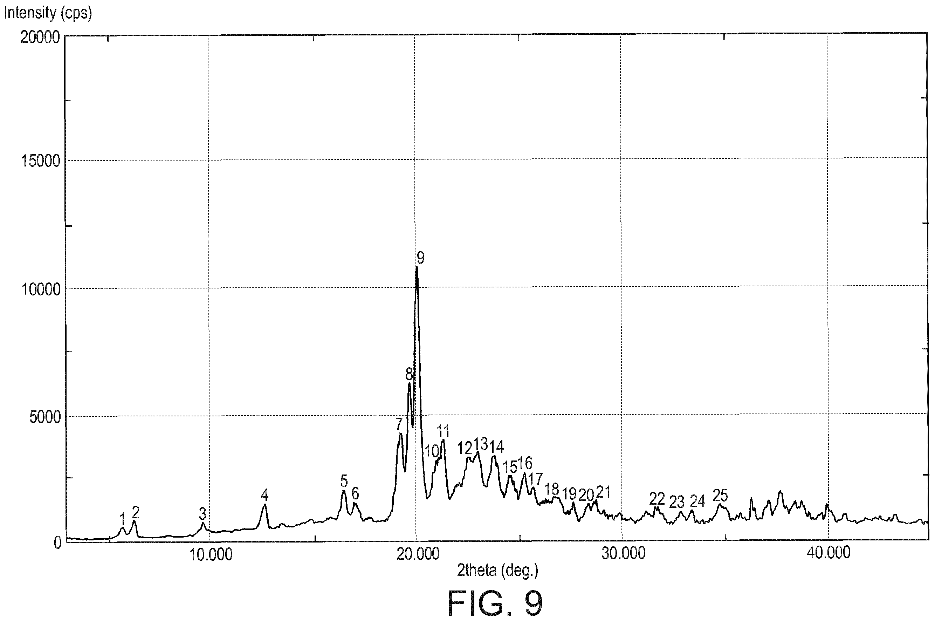

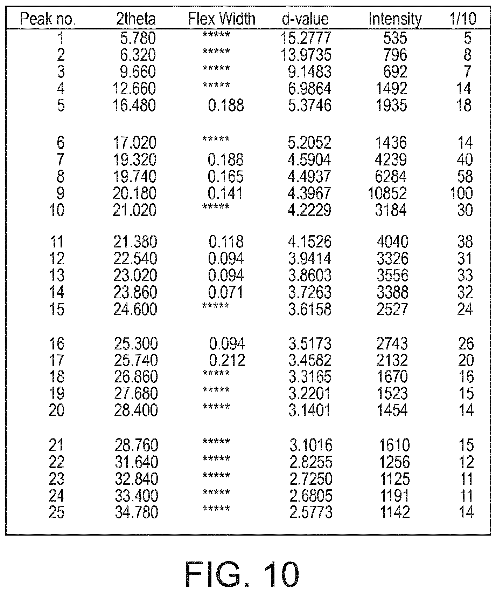

The importance of pure input API can be further illustrated by reference to Example 2, and associated Figures 7 to 12. Tablets (25 mg and 150 mg compositions as set out in Example 9) were stored at 40°C ± 2°C and 75% ± 5% relative humidity for a period of 3 months. However, the input API included trace amounts of erlotinib hydrochloride Form B according to Example 2. The X-ray powder diffraction pattern of the tablets in Figures 7 to 12 indicate transformation to Form B has occurred for both lowest and highest strength (25mg and 150mg) following 3 months of storage under accelerated conditions.

It should be pointed out that the provided results for the stability measurements were generated in the Examples based on a conventional X-ray powder diffraction scan and therefore the peak at 6.2 ± 0.2 degrees 2-theta was absent from the initial analysis results. Traces of Form B for both strengths were however then identified by visual observation of the X-ray powder diffraction pattern whereby a minute peak appears at the 2-theta value of 6.2 ± 0.2 on storage. Further to such observation, a slow scan X-ray powder diffraction was carried out (Example 2) for the API used for the manufacturing of the stability batches and indicated that traces of Form B were originally present in the API.

The solid pharmaceutical composition, obtained by the process according to the present invention, comprises erlotinib hydrochloride Form A, together with one or more selected pharmaceutically acceptable excipients, whereby such composition exhibits a dissolution profile of at least 40% at 30 minutes when measured in 1000 mL volume of 0.1N hydrochloric acid, at a temperature of 37°C and a paddle speed of 75 rpm. The dissolution is measured using USP-II apparatus (Paddle). Such dissolution profile is provided at least as a result of an input erlotinib hydrochloride API being of sufficient Form A purity, for example as determined by X-ray powder diffraction analysis as herein described. Preferably, the X-ray powder diffraction analysis is as herein described on a Bruker AXS D8 and with operating parameters also as herein described.

The erlotinib hydrochloride Form A, that includes no detectable erlotinib hydrochloride Form B by X-ray powder diffraction analysis has preferably a d₉₀ below about 25 microns, when measured with a Malvern Mastersizer 2000. More preferably, the d₉₀ is in the range of about 5 to 15 microns, and even more preferably in the range of about 9 to 14 microns.

The PSD of erlotinib hydrochloride Form A can be still further characterized by a d₅₀ in the range of about 1 to 6 microns, when measured with a Malvern Mastersizer 2000, and more preferably in the range of about 2 to 5 microns.

Still further, the PSD of erlotinib hydrochloride Form A can be further characterized by a d₁₀ in the range of about 0.5 to 2.5 microns, when measured with a Malvern Mastersizer 2000, and more preferably in the range of about 0.5 to 1.5 microns.

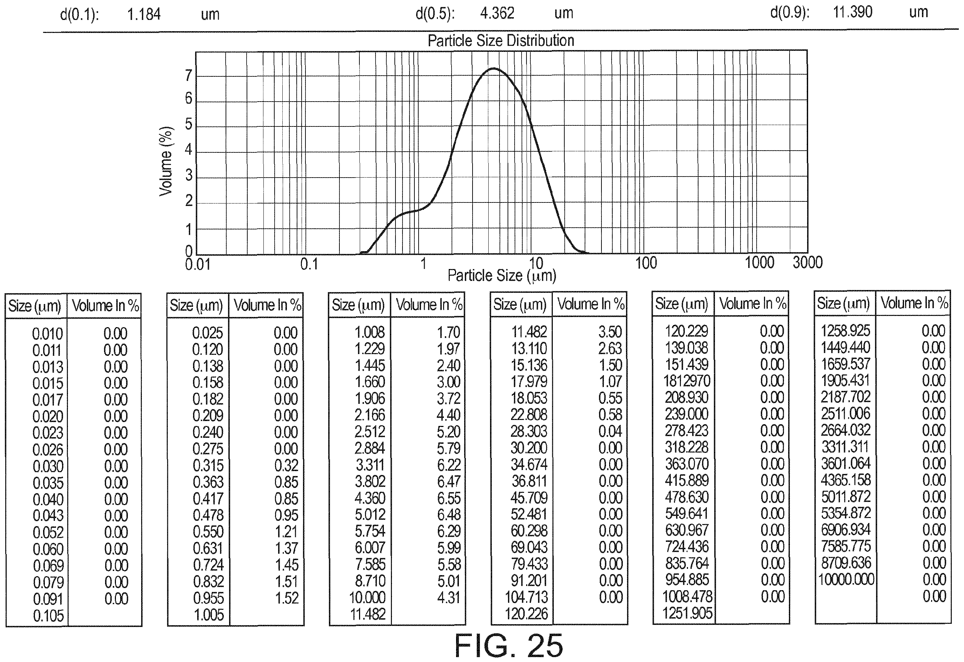

The erlotinib hydrochloride Form A, that includes no detectable erlotinib hydrochloride Form B when measured by X-ray powder diffraction analysis can further be characterized by a PSD as shown in Figure 25 as measured by a Malvern Mastersizer 2000.

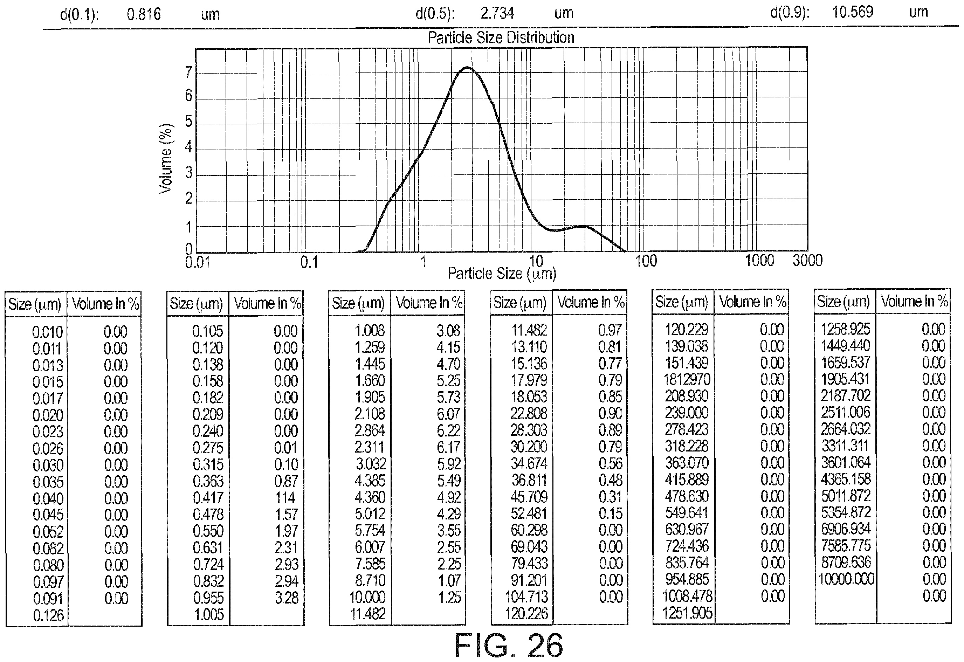

The erlotinib hydrochloride Form A, that includes no detectable erlotinib hydrochloride Form B when measured by X-ray powder diffraction analysis can further be characterized by a PSD as shown in Figure 26 as measured by a Malvern Mastersizer 2000.

The specific operating parameters of the Malvern Mastersizer 2000 as used in the measurements of the present invention are provided hereinafter in greater detail in the Experimental portion of the description.

The one or more excipients as used in a solid oral dosage form according to the present invention preferably include pharmaceutically acceptable excipients, such as fillers, binders, disintegrants, glidants, lubricants and the like.

Suitable fillers for inclusion in solid oral dosage forms according to the present invention include sugars, sugar alcohols and polymeric glycosides. Examples of sugars are sucrose, glucose and lactose as the monohydrate or in anhydrous form. Examples of sugar alcohols include mannitol, xylitol and sorbitol. Examples of polymeric glycosides are maltodextrin, microcrystalline cellulose and starches of different origins. Particularly suitable fillers include lactose and / or microcrystalline cellulose or the like.

Suitable binders for inclusion in solid oral dosage forms according to the present invention so as to ensure the required mechanical strength, include wet and / or dry binders depending on the formulation process employed. Typical binders include polymers, such as polyvinylpyrrolidone, and cellulose derivatives, such as hydroxypropyl cellulose and microcrystalline cellulose. In certain embodiments, a separate binder might not be necessary, and sufficient binding function might be provided by the dual excipient function of a filler excipient, such as microcrystalline cellulose.

Examples of suitable disintegrants for inclusion in solid oral dosage forms according to the present invention are crospovidone and croscarmellose, such as croscarmellose sodium, starches and modified starches, e.g. maize starch, in pregelatinized form or as sodium glycolate, and hydroxypropyl cellulose with a low degree of substitution (L-HPC). A preferred disintegrant is sodium starch glycolate.

Examples of suitable glidants, include colloidal silicon dioxide, calcium silicates and talcum.

Preferred lubricants include stearic acid or salts thereof, with a particularly preferred lubricant comprising magnesium stearate.

The following Figures, Experimental and Examples are provided to illustrate the invention and are not to be construed as limiting the scope of the invention in any manner.
**Figure 1a****:** X-ray powder diffraction pattern (raw data) of 150mg tablet according to Example 9 at time zero. ["Pure" Form A input API - Example 1]
**Figure 1b****:** X-ray powder diffraction pattern (peak search) of 150mg tablet according to Example 9 at time zero. ["Pure" Form A input API - Example 1]
**Figure 1c****:** Peak search results from Figure 1b. ["Pure" Form A input API - Example 1]
**Figure 2****:** X-ray powder diffraction pattern (raw data) of 150mg tablet according to Example 9 at 6 months storage at accelerated conditions, 40°C ± 2°C and 75% ± 5% relative humidity. ["Pure" Form A input API - Example 1]
**Figure 3****:** X-ray powder diffraction pattern (peak search) of 150mg tablet according to Example 9 at 6 months storage at accelerated conditions, 40°C ± 2°C and 75% ± 5% relative humidity. ["Pure" Form A input API - Example 1]
**Figure 4****:** Peak search results from Figure 3. ["Pure" Form A input API - Example 1]
**Figure 5****:** X-ray powder diffraction pattern of erlotinib hydrochloride of Example 1 ["Pure" Form A], and digitized patterns of erlotinib hydrochloride Forms A and B according to WO 01/34574.
**Figure 6****:** X-ray powder diffraction pattern of erlotinib hydrochloride of Example 1 ["Pure" Form A], the 2-theta region from 4-7 was scanned with a 20 times slower scan rate compared to Figure 5.
**Figure 7a****:** X-ray powder diffraction pattern (raw data) of 150mg tablet according to Example 9 at time zero. ["Impure" Form A input API - Example 2]
**Figure 7b****:** X-ray powder diffraction pattern (peak search) of 150mg tablet according to Example 9 at time zero. ["Impure" Form A input API - Example 2]
**Figure 7c****:** Peak search results from Figure 7b. ["Impure" Form A input API - Example 2]
**Figure 8**: X-ray powder diffraction pattern (raw data) of 150mg tablet according to Example 9 at 3 months storage at accelerated conditions, 40°C ± 2°C and 75% ± 5% relative humidity. ["Impure" Form A input API - Example 2]
**Figure 9**: X-ray powder diffraction pattern (peak search) of 150mg tablet according to Example 9 at 3 months storage at accelerated conditions, 40°C ± 2°C and 75% ± 5% relative humidity. ["Impure" Form A input API - Example 2]
**Figure 10****:** Peak search results from Figure 9. ["Impure" Form A input API - Example 2]
**Figure 11****:** X-ray powder diffraction pattern of erlotinib hydrochloride of Example 2 ["Impure" Form A], and digitized patterns of erlotinib hydrochloride Forms A and B according to WO 01/34574.
**Figure 12****:** X-ray powder diffraction pattern of erlotinib hydrochloride of Example 2 ["Impure" Form A], the 2-theta region from 4-7 was scanned with a 20 times slower scan rate compared to Figure 11.
**Figure 13****:** X-ray powder diffraction pattern of erlotinib hydrochloride of Example 3 ["Pure" Form A], and digitized patterns of erlotinib hydrochloride Forms A and B according to WO 01/34574.
**Figure 14****:** X-ray powder diffraction pattern of erlotinib hydrochloride of Example 3 ["Pure" Form A], the 2-theta region from 4-7 was scanned with a 13 times slower scan rate compared to Figure 13.
**Figure 15****:** X-ray powder diffraction pattern of erlotinib hydrochloride of Example 4 [present in a dry mixture] ["Pure" Form A], and digitized patterns of erlotinib hydrochloride Forms A and B according to WO 01/34574.
**Figure 16****:** X-ray powder diffraction pattern of erlotinib hydrochloride of Example 4 [present in a dry mixture] ["Pure" Form A], the 2-theta region from 4-7 was scanned with a 13 times slower scan rate compared to Figure 15.
**Figure 17****:** X-ray powder diffraction pattern of erlotinib hydrochloride of Example 5 [present in granules] ["Pure" Form A], and digitized patterns of erlotinib hydrochloride Forms A and B according to WO 01/34574.
**Figure 18****:** X-ray powder diffraction pattern of erlotinib hydrochloride of Example 5 [present in granules] ["Pure" Form A], the 2-theta region from 4-7 was scanned with a 13 times slower scan rate compared to Figure 17.
**Figure 19****:** X-ray powder diffraction pattern of erlotinib hydrochloride of Example 6 ["Impure" Form A], and digitized patterns of erlotinib hydrochloride Forms A and B according to WO 01/34574.
**Figure 20****:** X-ray powder diffraction pattern of erlotinib hydrochloride of Example 6 ["Impure" Form A], the 2-theta region from 4-7 was scanned with a 13 times slower scan rate compared to Figure 19.
**Figure 21****:** X-ray powder diffraction pattern of erlotinib hydrochloride of Example 7 [present in a dry mixture] ["Impure" Form A], and digitized patterns of erlotinib hydrochloride Forms A and B according to WO 01/34574.
**Figure 22****:** X-ray powder diffraction pattern of erlotinib hydrochloride of Example 7 [present in a dry mixture] ["Impure" Form A], the 2-theta region from 4-7 was scanned with a 13 times slower scan rate compared to Figure 21.
**Figure 23****:** X-ray powder diffraction pattern of erlotinib hydrochloride of Example 8 [present in granules] ["Impure" Form A], and digitized patterns of erlotinib hydrochloride Forms A and B according to WO 01/34574.
**Figure 24****:** X-ray powder diffraction pattern of erlotinib hydrochloride of Example 8 [present in granules] ["Impure" Form A], the 2-theta region from 4-7 was scanned with a 13 times slower scan rate compared to Figure 23.
**Figure 25****:** A PSD of erlotinib hydrochloride Form A.
**Figure 26****:** A PSD of erlotinib hydrochloride Form A.

### Experimental:

### X-ray Powder Diffractometer:

**Instrument type used:** Bruker AXS D8
**Source:** Cu Kα (λ=1.54°A)
**Detector type:** LynxEye
**Divergence slit:** 0.6 mm
**Voltage:** 40kV
**Current:** 40mA
**Background removal:** Yes using DIFFRAC plus EVA 12.0 software
**Start angle:** 3 2-theta for reference analysis, 4 2-theta for scan using conditions of the invention
**End angle:** 35 2-theta for reference analysis, 7 2-theta for scan using conditions of the invention
**Step size:** 0.02 2-theta
**Scanning rate:** 0.5 s / step for reference analysis, 4 s / step for scan using conditions of the invention
**Scan type:** Locked Coupled
**Scan mode:** Continuous
**Sample holder:** PMMA holder with a 25 mm diameter and 1.5 mm depth circular cavity for spreading the sample.
**Glass slides:** 25x75x1 mm glass slides
**Sample Preparation:** The sample was prepared by spreading the powder in sample holder with the help of a glass slide.

### Malvern Mastersizer:

### Principle:

### Laser Diffraction technique

### Wet dispersion method

**Equipment:** Malvern Mastersizer 2000
**Sample Handling Unit:** Hydro 2000 S (A)

### Example 1:

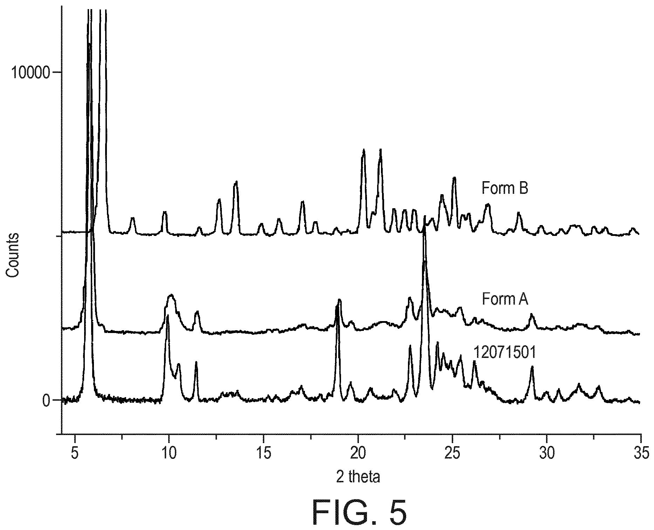

Erlotinib hydrochloride was analyzed by X-ray powder diffraction. The X-ray powder diffraction pattern along with the digitized patterns of erlotinib hydrochloride Forms A and B of WO 01/34574, can be found in Figure 5. The reflections are also set out in the following table.

| Angle 2-Theta ° | d value Angstrom | Intensity Count | Intensity % % |
|---|---|---|---|
| 5.622 | 15.70638 | 30266 | 100.0 |
| 9.802 | 9.01642 | 2633 | 8.7 |
| 10.366 | 8.52659 | 1136 | 3.8 |
| 11.303 | 7.82222 | 1196 | 4.0 |
| 12.717 | 6.95524 | 256 | 0.8 |
| 13.501 | 6.55322 | 329 | 1.1 |
| 15.189 | 5.82847 | 172 | 0.6 |
| 15.603 | 5.67463 | 173 | 0.6 |
| 16.471 | 5.37757 | 317 | 1.0 |
| 16.921 | 5.23544 | 459 | 1.5 |
| 17.940 | 4.94057 | 213 | 0.7 |
| 18.413 | 4.81467 | 224 | 0.7 |
| 18.881 | 4.69635 | 2994 | 9.9 |
| 19.569 | 4.53276 | 589 | 1.9 |
| 20.608 | 4.30650 | 404 | 1.3 |
| 21.885 | 4.05799 | 369 | 1.2 |
| 22.768 | 3.90264 | 1725 | 5.7 |
| 23.535 | 3.77707 | 5721 | 18.9 |
| 24.225 | 3.67096 | 1838 | 6.1 |
| 24.550 | 3.62317 | 1484 | 4.9 |
| 25.451 | 3.49684 | 1414 | 4.7 |
| 26.210 | 3.39732 | 1272 | 4.2 |
| 29.258 | 3.05000 | 1071 | 3.5 |
| 30.047 | 2.97168 | 311 | 1.0 |
| 30.691 | 2.91080 | 391 | 1.3 |

Based on the above data and the polymorph characterization of WO 01/34574, it is understood that the polymorph of Example 1 is erlotinib hydrochloride Form A. In order to confirm the absence of even trace amounts of erlotinib hydrochloride Form B, the 2-theta region from 4-7 was scanned with a 20 times slower scan rate and the results are shown in Figure 6. From Figure 6 it is apparent that the characteristic polymorph B peak at 6.3 2-theta is missing. This confirms that the polymorph of Example 1 is pure erlotinib hydrochloride Form A with not even trace amounts of Form B.

| Angle 2-Theta ° | d value Angstrom | Intensity Count | Intensity % % |
|---|---|---|---|
| 5.624 | 15.70178 | 406721 | 100.0 |

### Example 2:

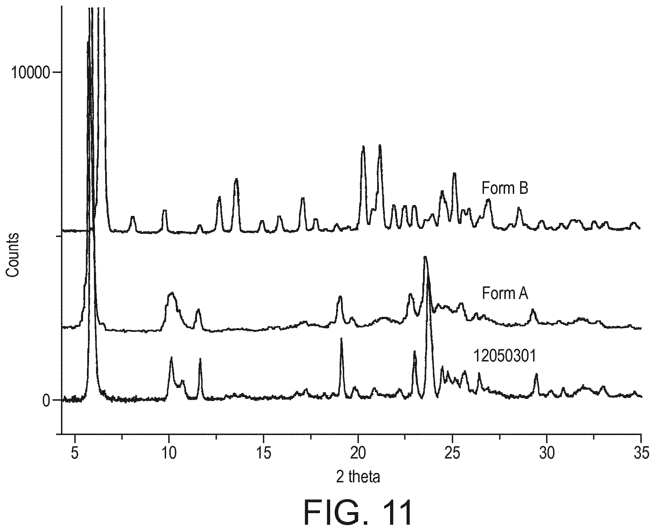

Erlotinib hydrochloride was analyzed by X-ray powder diffraction. The X-ray powder diffraction pattern along with the digitized patterns of erlotinib hydrochloride Forms A and B of WO 01/34574, can be found in Figure 11. The reflections are also set out in the following table.

| Angle 2-Theta ° | d value Angstrom | Intensity Count | Intensity % % |
|---|---|---|---|
| 5.642 | 15.65201 | 48183 | 100.0 |
| 6.201 | 14.24141 | 503 | 1.0 |
| 9.819 | 9.00048 | 2585 | 5.4 |
| 10.379 | 8.51634 | 1096 | 2.3 |
| 11.327 | 7.80541 | 2008 | 4.2 |
| 12.711 | 6.95876 | 194 | 0.4 |
| 13.173 | 6.71559 | 248 | 0.5 |
| 13.550 | 6.52976 | 281 | 0.6 |
| 16.476 | 5.37612 | 281 | 0.6 |
| 16.951 | 5.22638 | 470 | 1.0 |
| 17.996 | 4.92532 | 235 | 0.5 |
| 18.429 | 4.81051 | 221 | 0.5 |
| 18.913 | 4.68847 | 3134 | 6.5 |
| 19.578 | 4.53065 | 471 | 1.0 |
| 20.617 | 4.30463 | 410 | 0.9 |
| 21.975 | 4.04163 | 425 | 0.9 |
| 22.796 | 3.89780 | 2154 | 4.5 |
| 23.553 | 3.77420 | 6128 | 12.7 |
| 24.241 | 3.66869 | 1794 | 3.7 |
| 24.565 | 3.62094 | 1315 | 2.7 |
| 24.940 | 3.56745 | 1150 | 2.4 |
| 25.474 | 3.49377 | 1255 | 2.6 |
| 26.227 | 3.39515 | 1213 | 2.5 |
| 26.622 | 3.34574 | 551 | 1.1 |
| 29.272 | 3.04855 | 1182 | 2.5 |
| 30.072 | 2.96921 | 373 | 0.8 |
| 30.700 | 2.90996 | 398 | 0.8 |

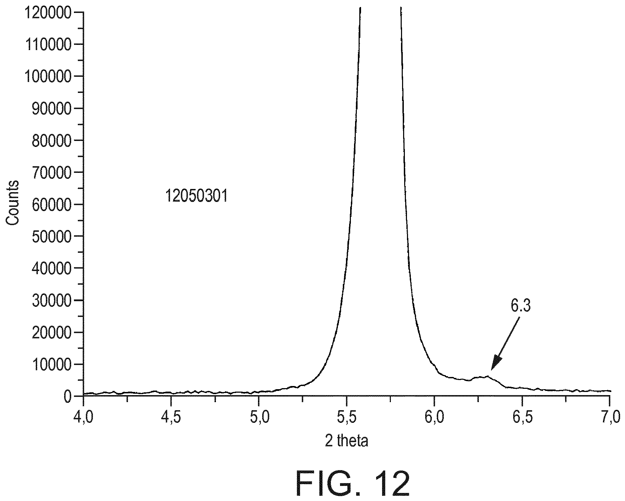

In order to confirm the absence / presence of even trace amounts of erlotinib hydrochloride Form B, the 2-theta region from 4-7 was scanned with a 20 times slower scan rate and the results are shown in Figure 12. From Figure 12 it is apparent that the characteristic polymorph B peak at 6.3 2-theta is present. This confirms that the polymorph of Example 2 is erlotinib hydrochloride Form A in admixture with Form B.

| Angle 2-Theta ° | d value Angstrom | Intensity Count | Intensity % % |
|---|---|---|---|
| 5.724 | 15.42667 | 531633 | 100.0 |
| 6.263 | 14.10168 | 4484 | 0.8 |

### Example 3:

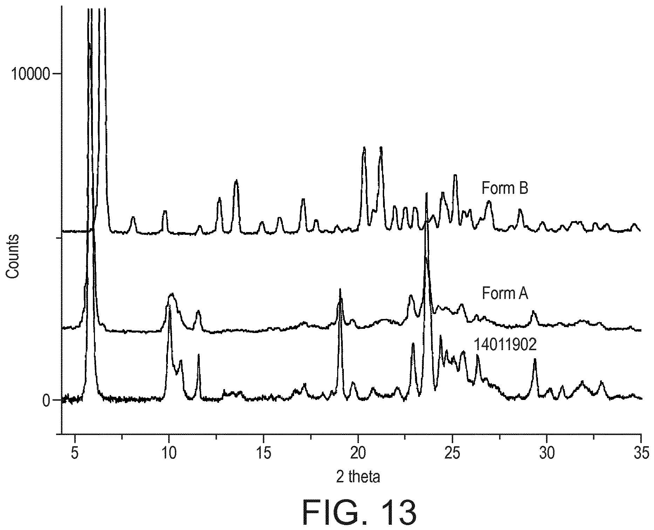

Erlotinib hydrochloride was analyzed by X-Ray powder diffraction. The X-Ray powder diffraction pattern along with the digitized patterns of polymorphs A and B of erlotinib hydrochloride Forms A and B of WO 01/34574, can be found in Figure 13. The reflections are also set out in the following table.

| Angle 2-Theta ° | d value Angstrom | Intensity Count | Intensity % % |
|---|---|---|---|
| 5.675 | 15.56013 | 39879 | 100.0 |
| 9.864 | 8.95938 | 2923 | 7.3 |
| 10.411 | 8.48982 | 1209 | 3.0 |
| 11.375 | 7.77279 | 1413 | 3.5 |
| 12.770 | 6.92688 | 310 | 0.8 |
| 13.225 | 6.68923 | 228 | 0.6 |
| 13.610 | 6.50103 | 261 | 0.7 |
| 16.534 | 5.35719 | 310 | 0.8 |
| 17.002 | 5.21087 | 467 | 1.2 |
| 18.943 | 4.68100 | 3398 | 8.5 |
| 19.603 | 4.52501 | 526 | 1.3 |
| 20.686 | 4.29029 | 349 | 0.9 |
| 21.961 | 4.04411 | 348 | 0.9 |
| 22.826 | 3.89270 | 1728 | 4.3 |
| 23.597 | 3.76726 | 6325 | 15.9 |
| 24.303 | 3.65944 | 1965 | 4.9 |
| 24.608 | 3.61479 | 1509 | 3.8 |
| 24.997 | 3.55943 | 1321 | 3.3 |
| 25.507 | 3.48938 | 1468 | 3.7 |
| 25.885 | 3.43928 | 573 | 1.4 |
| 26.297 | 3.38635 | 1322 | 3.3 |
| 26.677 | 3.33888 | 636 | 1.6 |
| 27.124 | 3.28493 | 429 | 1.1 |
| 27.366 | 3.25634 | 351 | 0.9 |
| 29.303 | 3.04541 | 1192 | 3.0 |
| 30.126 | 2.96409 | 294 | 0.7 |
| 30.764 | 2.90398 | 384 | 1.0 |
| 31.411 | 2.84565 | 226 | 0.6 |
| 31.827 | 2.80945 | 546 | 1.4 |
| 32.848 | 2.72437 | 478 | 1.2 |

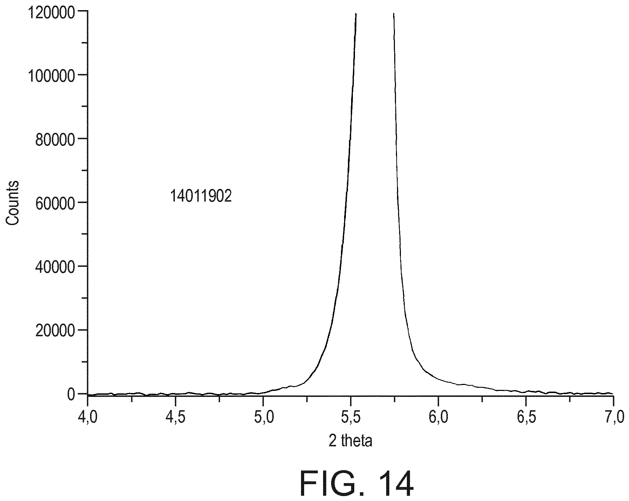

Based on the above data and the polymorph characterization of WO 01/34574, it is understood that the polymorph of Example 3 is erlotinib hydrochloride Form A. In order to confirm the absence of even trace amounts of erlotinib hydrochloride Form B, the 2-theta region from 4-7 was scanned with a 13 times slower scan rate and the results are shown in Figure 14. From Figure 14 it is apparent that the characteristic polymorph B peak at 6.3 2-theta is missing. This confirms that the polymorph of Example 3 is pure erlotinib hydrochloride Form A with not even trace amounts of Form B.

| Angle 2-Theta ° | d value Angstrom | Intensity Count | Intensity % % |
|---|---|---|---|
| 5.679 | 15.54873 | 533230 | 100.0 |

### Example 4:

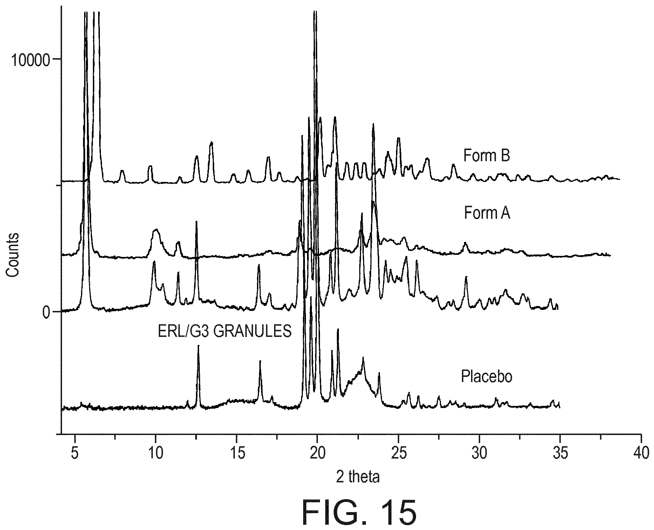

Erlotinib hydrochloride dry mixture was analyzed by X-Ray powder diffraction. The X-Ray powder diffraction pattern along with the digitized patterns of polymorphs A and B of erlotinib hydrochloride Forms A and B of WO 01/34574, and the placebo pattern, can be found in Figure 15. The reflections are also set out in the following table.

| Angle 2-Theta ° | d value Angstrom | Intensity Count | Intensity % % |
|---|---|---|---|
| 5.671 | 15.57269 | 65978 | 79.6 |
| 9.825 | 8.99499 | 5901 | 7.1 |
| 10.381 | 8.51501 | 3158 | 3.8 |
| 11.330 | 7.80320 | 4536 | 5.5 |
| 11.824 | 7.47848 | 1381 | 1.7 |
| 12.494 | 7.07907 | 10746 | 13.0 |
| 13.169 | 6.71786 | 998 | 1.2 |
| 13.575 | 6.51782 | 1104 | 1.3 |
| 16.384 | 5.40583 | 5394 | 6.5 |
| 17.029 | 5.20273 | 1949 | 2.4 |
| 18.927 | 4.68486 | 10734 | 13.0 |
| 19.129 | 4.63590 | 21046 | 25.4 |
| 19.580 | 4.53013 | 23248 | 28.0 |
| 20.000 | 4.43589 | 82881 | 100.0 |
| 20.837 | 4.25967 | 6853 | 8.3 |
| 21.224 | 4.18278 | 17683 | 21.3 |
| 22.004 | 4.03630 | 2441 | 2.9 |
| 22.809 | 3.89569 | 11648 | 14.1 |
| 23.576 | 3.77057 | 22433 | 27.1 |
| 24.281 | 3.66263 | 5870 | 7.1 |
| 24.594 | 3.61679 | 4770 | 5.8 |
| 24.989 | 3.56056 | 3968 | 4.8 |
| 25.551 | 3.48348 | 6328 | 7.6 |
| 26.229 | 3.39489 | 5886 | 7.1 |
| 26.584 | 3.35037 | 2150 | 2.6 |
| 27.034 | 3.29561 | 1260 | 1.5 |
| 27.448 | 3.24686 | 1590 | 1.9 |
| 28.192 | 3.16281 | 803 | 1.0 |
| 28.498 | 3.12958 | 1017 | 1.2 |

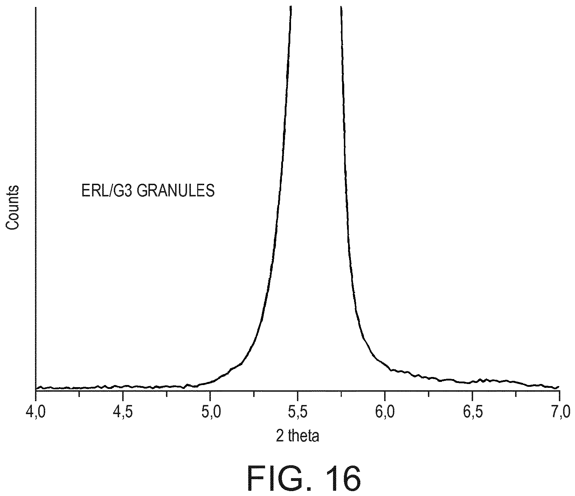

Based on the above data and the polymorph characterization of WO 01/34574, it is understood that the polymorph of the dry mixture of Example 4 is erlotinib hydrochloride Form A. In order to confirm the absence of even trace amounts of erlotinib hydrochloride Form B, the 2-theta region from 4-7 was scanned with a 13 times slower scan rate and the results are shown in Figure 16. From Figure 16 it is apparent that the characteristic polymorph B peak at 6.3 2-theta is missing. This confirms that the polymorph of Example 4 is pure erlotinib hydrochloride Form A with not even trace amounts of Form B.

| Angle 2-Theta ° | d value Angstrom | Intensity Count | Intensity % % |
|---|---|---|---|
| 5.652 | 15.62454 | 664846 | 100.0 |

### Example 5:

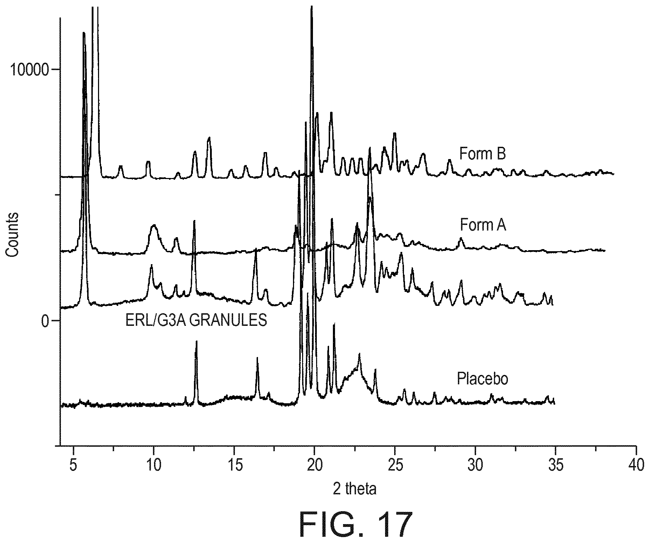

Erlotinib hydrochloride granules was analyzed by X-Ray powder diffraction. The X-Ray powder diffraction pattern along with the digitized patterns of polymorphs A and B of erlotinib hydrochloride Forms A and B of WO 01/34574, and the placebo pattern, can be found in Figure 17. The reflections are also set out in the following table.

| Angle 2-Theta ° | d value Angstrom | Intensity Count | Intensity % % |
|---|---|---|---|
| 5.606 | 15.75207 | 23002 | 88.9 |
| 7.870 | 11.22521 | 612 | 2.4 |
| 8.120 | 10.87935 | 897 | 3.5 |
| 8.531 | 10.35604 | 843 | 3.3 |
| 8.769 | 10.07652 | 896 | 3.5 |
| 9.021 | 9.79453 | 925 | 3.6 |
| 9.763 | 9.05191 | 4723 | 18.2 |
| 10.301 | 8.58083 | 2841 | 11.0 |
| 11.260 | 7.85186 | 2729 | 10.5 |
| 11.750 | 7.52530 | 1834 | 7.1 |
| 12.433 | 7.11345 | 7817 | 30.2 |
| 12.747 | 6.93930 | 1919 | 7.4 |
| 13.131 | 6.73684 | 1781 | 6.9 |
| 13.469 | 6.56859 | 1684 | 6.5 |
| 14.329 | 6.17633 | 1339 | 5.2 |
| 14.690 | 6.02523 | 1048 | 4.0 |
| 15.150 | 5.84336 | 898 | 3.5 |
| 15.588 | 5.68027 | 740 | 2.9 |
| 16.313 | 5.42925 | 9948 | 38.4 |
| 16.974 | 5.21947 | 2117 | 8.2 |
| 18.908 | 4.68963 | 9721 | 37.5 |
| 19.064 | 4.65166 | 14216 | 54.9 |
| 19.525 | 4.54273 | 16167 | 62.5 |
| 19.925 | 4.45258 | 25888 | 100.0 |
| 20.759 | 4.27548 | 6181 | 23.9 |
| 21.185 | 4.19038 | 12766 | 49.3 |
| 21.970 | 4.04249 | 2417 | 9.3 |
| 22.723 | 3.91013 | 9215 | 35.6 |
| 23.524 | 3.77884 | 18196 | 70.3 |
| 24.216 | 3.67241 | 5032 | 19.4 |
| 24.536 | 3.62523 | 4304 | 16.6 |
| 24.929 | 3.56894 | 3880 | 15.0 |
| 25.501 | 3.49015 | 7802 | 30.1 |
| 26.172 | 3.40212 | 4178 | 16.1 |
| 26.512 | 3.35930 | 2119 | 8.2 |
| 26.780 | 3.32632 | 1698 | 6.6 |

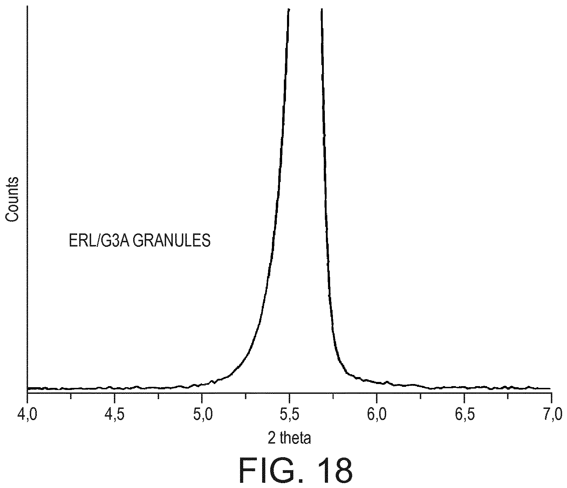

Based on the above data and the polymorph characterization of WO 01/34574, it is understood that the polymorph of the granules of Example 5 is erlotinib hydrochloride Form A. In order to confirm the absence of even trace amounts of erlotinib hydrochloride Form B, the 2-theta region from 4-7 was scanned with a 13 times slower scan rate and the results are shown in Figure 18. From Figure 18 it is apparent that the characteristic polymorph B peak at 6.3 2-theta is missing. This confirms that the polymorph of Example 4 is pure erlotinib hydrochloride Form A with not even trace amounts of Form B.

| Angle 2-Theta ° | d value Angstrom | Intensity Count | Intensity % % |
|---|---|---|---|
| 5.608 | 15.74628 | 232544 | 100.0 |

### Example 6:

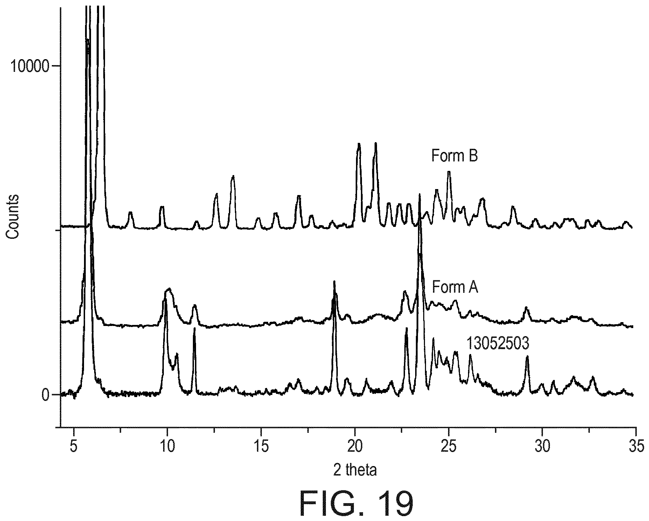

Erlotinib hydrochloride was analyzed by X-Ray powder diffraction. The X-Ray powder diffraction pattern along with the digitized patterns of polymorphs A and B of erlotinib hydrochloride Forms A and B of WO 01/34574, can be found in Figure 19. The reflections are also set out in the following table.

| Angle | d value | Intensity | Intensity % |
|---|---|---|---|
| 2-Theta ° | Angstrom | Count | % |
| 5.664 | 15.59187 | 53897 | 100.0 |
| 6.195 | 14.25597 | 464 | 0.9 |
| 9.814 | 9.00494 | 2926 | 5.4 |
| 10.384 | 8.51260 | 1263 | 2.3 |
| 11.349 | 7.79019 | 2029 | 3.8 |
| 13.576 | 6.51702 | 243 | 0.5 |
| 15.228 | 5.81352 | 170 | 0.3 |
| 15.627 | 5.66604 | 110 | 0.2 |
| 16.495 | 5.36989 | 342 | 0.6 |
| 16.965 | 5.22197 | 472 | 0.9 |
| 17.971 | 4.93189 | 188 | 0.3 |
| 18.433 | 4.80943 | 220 | 0.4 |
| 18.912 | 4.68859 | 3489 | 6.5 |
| 19.574 | 4.53163 | 493 | 0.9 |
| 20.627 | 4.30258 | 484 | 0.9 |
| 21.963 | 4.04381 | 378 | 0.7 |
| 22.804 | 3.89650 | 2039 | 3.8 |
| 23.560 | 3.77308 | 6201 | 11.5 |
| 24.260 | 3.66585 | 1738 | 3.2 |
| 24.571 | 3.62005 | 1347 | 2.5 |
| 25.445 | 3.49772 | 1290 | 2.4 |
| 26.238 | 3.39382 | 1290 | 2.4 |
| 29.278 | 3.04796 | 1154 | 2.1 |
| 30.089 | 2.96758 | 317 | 0.6 |
| 30.711 | 2.90888 | 409 | 0.8 |
| 31.536 | 2.83471 | 296 | 0.6 |
| 31.797 | 2.81201 | 557 | 1.0 |
| 32.837 | 2.72529 | 545 | 1.0 |
| 34.508 | 2.59705 | 170 | 0.3 |

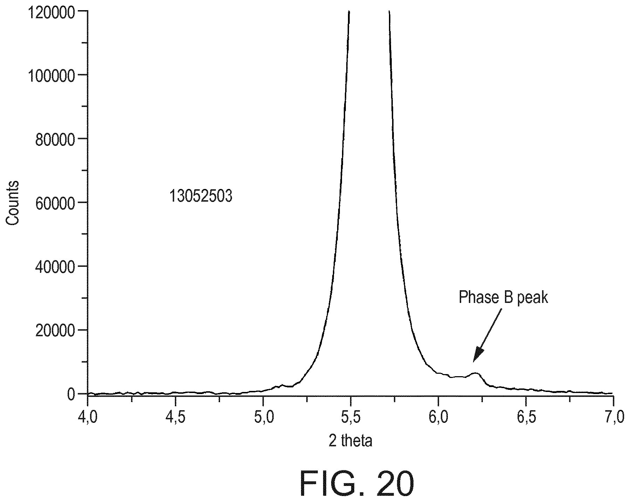

Based on the above data and the polymorph characterization of WO 01/34574, it is understood that the polymorph of Example 6 is erlotinib hydrochloride Form A. In order to detect possible presence of erlotinib hydrochloride Form B, the 2-theta region from 4-7 was scanned with a 13 times slower scan rate and the results are shown in Figure 20. From Figure 20, it is apparent that the characteristic polymorph B peak at 6.3 2-theta is present so API is mainly polymorph A, but presence of minor quantity of Form B (approx. 0.5 %) was detected.

| Angle | d value | Intensity | Intensity % |
|---|---|---|---|
| 2-Theta ° | Angstrom | Count | % |
| 5.660 | 15.60284 | 725427 | 100.0 |
| 6.183 | 14.28391 | 5965 | 0.8 |

### Example 7:

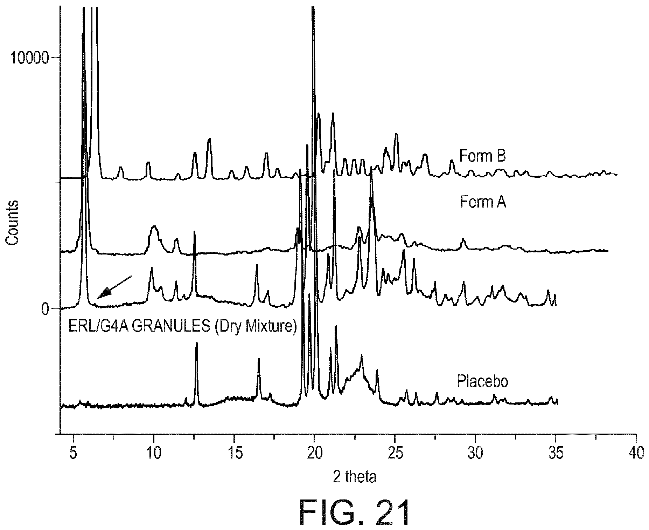

Erlotinib hydrochloride dry mixture was analyzed by X-Ray powder diffraction. The X-Ray powder diffraction pattern along with the digitized patterns of polymorphs A and B of erlotinib hydrochloride Forms A and B of WO 01/34574, and the placebo pattern, can be found in Figure 21. The reflections are also set out in the following table.

| Angle | d value | Intensity | Intensity % |
|---|---|---|---|
| 2-Theta ° | Angstrom | Count | % |
| 5.638 | 15.66249 | 43252 | 75.6 |
| 6.181 | 14.28666 | 371 | 0.6 |
| 9.796 | 9.02164 | 4723 | 8.3 |
| 10.358 | 8.53382 | 2469 | 4.3 |
| 11.316 | 7.81348 | 3056 | 5.3 |
| 11.794 | 7.49738 | 1514 | 2.6 |
| 12.479 | 7.08774 | 9141 | 16.0 |
| 13.510 | 6.54881 | 1279 | 2.2 |
| 14.682 | 6.02841 | 574 | 1.0 |
| 16.350 | 5.41706 | 4568 | 8.0 |
| 17.017 | 5.20634 | 1775 | 3.1 |
| 18.908 | 4.68963 | 9452 | 16.5 |
| 19.107 | 4.64130 | 16544 | 28.9 |
| 19.540 | 4.53947 | 19454 | 34.0 |
| 19.972 | 4.44215 | 57202 | 100.0 |
| 20.794 | 4.26834 | 6267 | 11.0 |
| 21.211 | 4.18544 | 16460 | 28.8 |
| 21.949 | 4.04633 | 2025 | 3.5 |
| 22.761 | 3.90376 | 8352 | 14.6 |
| 23.546 | 3.77536 | 16771 | 29.3 |
| 24.244 | 3.66816 | 4461 | 7.8 |
| 24.545 | 3.62391 | 3873 | 6.8 |
| 24.944 | 3.56678 | 3373 | 5.9 |
| 25.539 | 3.48501 | 6776 | 11.8 |
| 26.192 | 3.39958 | 5690 | 9.9 |
| 26.566 | 3.35260 | 1841 | 3.2 |
| 27.451 | 3.24646 | 2866 | 5.0 |
| 28.132 | 3.16942 | 1300 | 2.3 |
| 28.458 | 3.13390 | 996 | 1.7 |
| 28.957 | 3.08101 | 1230 | 2.2 |
| 29.257 | 3.05010 | 2840 | 5.0 |

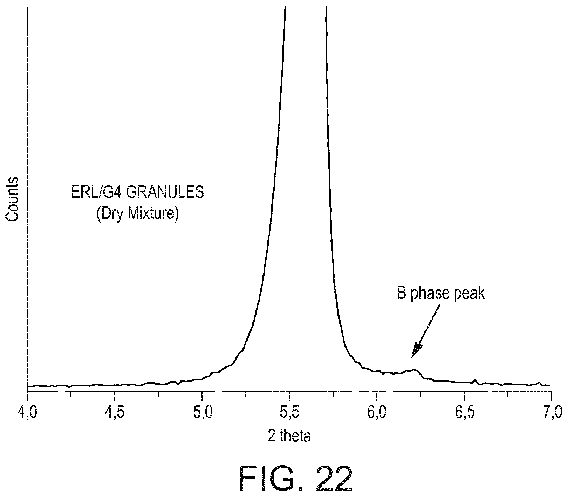

Based on the above data, the polymorph characterization of WO 01/34574 and Figure 21, it is understood that the polymorph of the dry mixture of Example 7 is mainly Form A but presence of minor quantity of Form B is also detected. In order to verify the possible presence of erlotinib hydrochloride Form B, the 2-theta region from 4-7 was scanned with a 13 times slower scan rate and the results are shown in Figure 22. The characteristic Form B peak was clearly observed. The relative intensity of this peak indicates that the percentage of Form B in API is approximately 0.4%.

| Angle | d value | Intensity | Intensity % |
|---|---|---|---|
| 2-Theta ° | Angstrom | Count | % |
| 5.639 | 15.66080 | 436478 | 100.0 |
| 6.188 | 14.27176 | 3634 | 0.8 |

### Example 8:

Erlotinib hydrochloride granules was analyzed by X-Ray powder diffraction. The X-Ray powder diffraction pattern along with the digitized patterns of polymorphs A and B of erlotinib hydrochloride

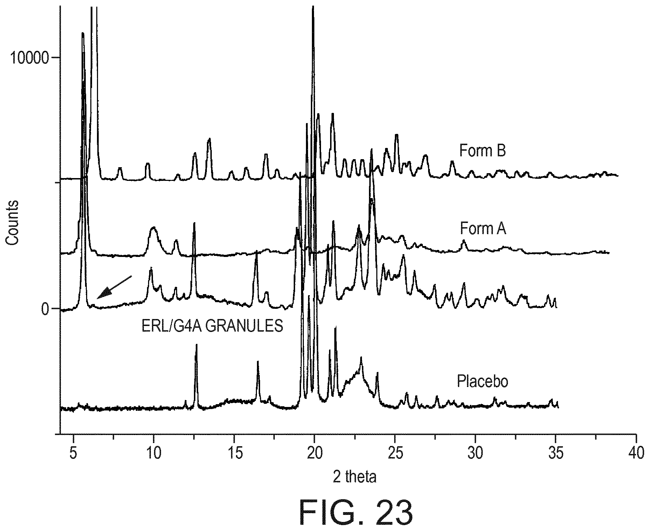

Forms A and B of WO 01/34574, and the placebo pattern, can be found in Figure 23. The reflections are also set out in the following table.

| Angle | d value | Intensity | Intensity % |
|---|---|---|---|
| 2-Theta ° | Angstrom | Count | % |
| 5.631 | 15.68160 | 27106 | 69.7 |
| 6.166 | 14.32165 | 546 | 1.4 |
| 8.152 | 10.83733 | 685 | 1.8 |
| 9.800 | 9.01831 | 4968 | 12.8 |
| 10.354 | 8.53721 | 2805 | 7.2 |
| 11.302 | 7.82246 | 2519 | 6.5 |
| 11.812 | 7.48635 | 1823 | 4.7 |
| 12.458 | 7.09930 | 10189 | 26.2 |
| 13.131 | 6.73686 | 1608 | 4.1 |
| 13.511 | 6.54858 | 1528 | 3.9 |
| 14.330 | 6.17587 | 1097 | 2.8 |
| 14.726 | 6.01076 | 839 | 2.2 |
| 15.184 | 5.83047 | 653 | 1.7 |
| 15.619 | 5.66883 | 617 | 1.6 |
| 16.369 | 5.41091 | 6863 | 17.7 |
| 16.994 | 5.21317 | 1963 | 5.1 |
| 18.923 | 4.68584 | 9683 | 24.9 |
| 19.105 | 4.64172 | 15270 | 39.3 |
| 19.555 | 4.53588 | 22054 | 56.7 |
| 19.959 | 4.44497 | 38864 | 100.0 |
| 20.815 | 4.26404 | 7631 | 19.6 |
| 21.176 | 4.19214 | 10397 | 26.8 |
| 21.981 | 4.04050 | 2377 | 6.1 |
| 22.766 | 3.90295 | 9941 | 25.6 |
| 23.552 | 3.77436 | 18933 | 48.7 |
| 24.241 | 3.66869 | 5224 | 13.4 |
| 24.551 | 3.62309 | 4488 | 11.5 |
| 24.946 | 3.56655 | 3875 | 10.0 |
| 25.525 | 3.48693 | 6327 | 16.3 |
| 26.210 | 3.39735 | 4375 | 11.3 |
| 26.547 | 3.35501 | 2035 | 5.2 |

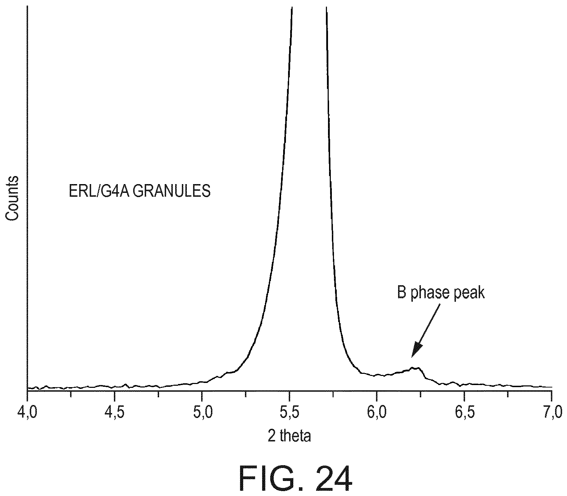

Based on the above data and the polymorph characterization of WO 01/34574, it is understood that the polymorph of the granules of Example 8 is mainly polymorph A but presence of minor quantity of Phase B was detected. In order to verify the possible presence of erlotinib hydrochloride Form B, the 2-theta region from 4-7 was scanned with a 13 times slower scan rate and the results are shown in Figure 24. From Figure 24 the characteristic erlotinib hydrochloride Form B peak is clearly observed. The relative intensity of this peak indicates that the percentage of phase B in API is approximately 0.9 %.

| Angle | d value | Intensity | Intensity % |
|---|---|---|---|
| 2-Theta ° | Angstrom | Count | % |
| 5.633 | 15.67701 | 272164 | 100.0 |
| 6.182 | 14.28439 | 4575 | 1.7 |

### Example 9:

The following tablet compositions were prepared, in particular using pure erlotinib hydrochloride Form A as above as input API.

| Ingredients | Function | **150 mg** | **100 mg** | **25 mg** |
|---|---|---|---|---|
| | | Qty. Per Tablet | | |
| | | mg | mg | mg |
| **Core tablets** | | | | |
| Erlotinib Hydrochloride¹ | Active | 163.80 | 109.20 | 27.30 |
| Lactose Monohydrate | Diluent | 183.6 | 122.4 | 30.6 |
| Microcrystalli ne cellulose- | Diluent | 92.4 | 61.6 | 15.4 |
| Sodium starch glycolate | Disintegrant | 9.0 | 6.0 | 1.5 |
| Isopropanol² | Granulation solvent | q.s. | q.s. | q.s. |
| Magnesium stearate | Lubricant | 7.2 | 4.8 | 1.2 |
| Total core weight | | 456.0 | 304.0 | 76.0 |

| **Film Coating** | | | | |
|---|---|---|---|---|
| Film coating | Coating agent | 18.0 | 12.0 | 3.0 |
| **Film-coated tablets** | | 474.0 | 316.0 | 79.0 |

| | | | | |
|---|---|---|---|---|
| ¹ The actual quantity of erlotinib hydrochloride was calculated according to the assay and the water content of the drug substance lot being used. The quantity of lactose monohydrate was adjusted accordingly. ² Solvent used in granulation process; evaporated during the process and not present in the final product. | | | | |

### Example 10:

The following PSD measurement was carried out.

### Preparation of solutions:

**Solution A:** Accurately weigh 27.22g of monopotassium phosphate, dissolve in 1000 ml of water, and mix well.

**Solution B:** Accurately transfer 11.2 ml of saturated sodium hydroxide, dilute to 1000ml with water, and mix well

**Buffer pH 7.4:** Solution A - Solution B (50 : 39.1), mix well. Adjust pH to 7.4 with diluted phosphoric acid.

**Sample solution:** 200 mg of homogeneous erlotinib hydrochloride sample was mixed with Tween 80 (2 drops) and buffer pH 7.4 (4 drops) to obtain paste.

### Procedure:

Refer to DQC-SOP 29A Use and Maintenance of Malvern Mastersizer 2000 and set the instrument parameters.

Add the sample solution to Hydro 2000 S(A) until the obscuration value lies within 10 - 20%.

Measure at 2500 rpm and 70% ultrasound (US) in Mastersizer dispersion cell after 4 minutes.

## Claims

1. A method of monitoring polymorphic purity of an erlotinib hydrochloride sample, the method comprising an X-ray powder diffraction analysis that includes a differentiation resolution and a 2-theta scan region that enables detection of the absence, or presence, of a characteristic 2-theta peak of erlotinib hydrochloride Form B in the region of 6.0 to 6.4 degrees 2-theta, which has a detection limit that enables detection of the presence of equal to, or less than, 0.2% erlotinib hydrochloride Form B in a test sample, wherein the X-ray powder diffraction analysis is operated at a current of 40 mA, a voltage of 40 kV, a step size of 0.02 degrees 2-theta and a time per step of 4.0 seconds, and with a divergence slit of 0.6 mm.

2. A method according to claim 1, which includes a differentiation resolution between adjacent 2-theta peaks of not more than about 0.1 degrees 2-theta.

3. A method according to any of claims 1 to 2, which employs a Bruker AXS D8, with a radiation source of Cu Kα (λ=1.54 Å) and a detector type LynxEye.

4. A method according to any of claims 1 to 3, carried out at any of the following stages of formulation development: as analysis of the API *per se*; as analysis of a formulation mixture to be granulated; as analysis of granules; and / or as analysis of a final dosage form.

5. A process for preparing a pharmaceutical composition comprising erlotinib hydrochloride Form A, and at least one pharmaceutically acceptable carrier, comprising:
obtaining a batch of erlotinib hydrochloride Form A;
analyzing the batch for the presence of erlotinib hydrochloride Form B so as to ensure erlotinib hydrochloride by carrying out a process according to any of claims 1 to 4;
preparing a pharmaceutical composition from the batch only if the batch is determined to have the required polymorphic purity of erlotinib hydrochloride Form A, whereby there is no detectable erlotinib hydrochloride Form B when measured by carrying out a process according to any of claims 1 to 4.

6. A process for preparing a packaged pharmaceutical composition comprising erlotinib hydrochloride Form A, comprising:
obtaining a pharmaceutical composition comprising erlotinib hydrochloride Form A;
analyzing the pharmaceutical composition for the presence of erlotinib hydrochloride Form B so as to ensure erlotinib hydrochloride by carrying out a process according to any of claims 1 to 4; and
packaging the pharmaceutical composition only if the erlotinib hydrochloride is determined to have the required polymorphic purity of erlotinib hydrochloride Form A, whereby there is no detectable erlotinib hydrochloride Form B when measured by carrying out a process according to any of claims 1 to 4.

7. A process of distributing a validated batch of a pharmaceutical composition comprising erlotinib hydrochloride Form A and at least one pharmaceutically acceptable carrier, comprising:
obtaining a batch of the pharmaceutical composition;
performing stability testing with a sample of the batch;
analyzing the sample of the batch for the presence of erlotinib hydrochloride Form B after said stability testing, so as to ensure erlotinib hydrochloride by carrying out a process according to any of claims 1 to 4;
validating the batch for distribution only if the sample of the batch after stability testing is determined to have the required polymorphic purity of erlotinib hydrochloride Form A,
whereby there is no detectable erlotinib hydrochloride Form B when measured by carrying out a process according to any of claims 1 to 4; and
distributing the validated batch.

## Patentansprüche

1. Verfahren zur Überwachung der polymorphen Reinheit einer Erlotinib-Hydrochlorid-Probe, wobei das Verfahren eine Röntgen-Pulverdiffraktionsanalyse umfasst, die eine Differenzierungsauflösung und einen 2-Theta-Scanbereich einschließt, der den Nachweis des Fehlens oder des Vorhandens eines charakteristischen 2-Theta-Peaks von Erlotinib-Hydrochlorid Form B im Bereich von 6,0 bis 6,4 Grad 2-theta ermöglicht, der eine Nachweisgrenze aufweist, die den Nachweis des Vorhandenseins von gleich oder weniger als 0,2 % Erlotinibhydrochlorid Form B in einer Testprobe ermöglicht, wobei die Röntgenpulverbeugungsanalyse bei einem Strom von 40 mA, einer Spannung von 40 kV, einer Schrittgröße von 0,02 Grad 2-theta und einer Zeit pro Schritt von 4,0 Sekunden und mit einem Divergenzspalt von 0,6 mm betrieben wird.

2. Verfahren nach Anspruch 1, das eine Differenzierungsauflösung zwischen benachbarten 2-theta-Peaks von nicht mehr als etwa 0,1 Grad 2-theta aufweist.

3. Verfahren nach einem der Ansprüche 1 bis 2, bei dem ein Bruker AXS D8 mit einer Cu-Ka-Strahlungsquelle (λ=1,54 Ä) und einem Detektor vom Typ LynxEye verwendet wird.

4. Verfahren nach einem der Ansprüche 1 bis 3, das in einem der folgenden Stadien der Formulierungsentwicklung durchgeführt wird: als Analyse des Wirkstoffs an sich; als Analyse einer zu granulierenden Formulierungsmischung; als Analyse von Granulat; und/oder als Analyse einer endgültigen Darreichungsform.

5. Verfahren zur Herstellung einer pharmazeutischen Zusammensetzung, die Erlotinib-Hydrochlorid Form A und mindestens einen pharmazeutisch akzeptablen Träger enthält, umfassend:
Gewinnung einer Charge von Erlotinib-Hydrochlorid Form A;
Analysieren der Charge auf das Vorhandensein von Erlotinib-Hydrochlorid Form B, um Erlotinib-Hydrochlorid sicherzustellen, indem ein Verfahren nach einem der Ansprüche 1 bis 4 durchgeführt wird;
Herstellen einer pharmazeutischen Zusammensetzung aus der Charge nur dann, wenn festgestellt wird, dass die Charge die erforderliche polymorphe Reinheit von Erlotinib-Hydrochlorid Form A aufweist, wobei kein Erlotinib-Hydrochlorid Form B nachweisbar ist, wenn es durch Ausführen eines Verfahrens nach einem der Ansprüche 1 bis 4 gemessen wird.

6. Verfahren zur Herstellung einer verpackten pharmazeutischen Zusammensetzung, die Erlotinibhydrochlorid Form A enthält, umfassend:
Gewinnung einer pharmazeutischen Zusammensetzung, die Erlotinib-Hydrochlorid Form A enthält;
Analysieren der pharmazeutischen Zusammensetzung auf das Vorhandensein von Erlotinib-Hydrochlorid Form B, um Erlotinib-Hydrochlorid sicherzustellen, indem ein Verfahren nach einem der Ansprüche 1 bis 4 durchgeführt wird; und
Verpacken der pharmazeutischen Zusammensetzung nur dann, wenn festgestellt wird, dass das Erlotinib-Hydrochlorid die erforderliche polymorphe Reinheit von Erlotinib-Hydrochlorid Form A aufweist, wodurch kein Erlotinib-Hydrochlorid Form B nachweisbar ist, wenn es durch Ausführen eines Verfahrens nach einem der Ansprüche 1 bis 4 gemessen wird.

7. Verfahren zum Vertrieb einer validierten Charge einer pharmazeutischen Zusammensetzung, die Erlotinib-Hydrochlorid Form A und mindestens einen pharmazeutisch akzeptablen Träger enthält, umfassend:
Gewinnung einer Charge der pharmazeutischen Zusammensetzung; Durchführung von Stabilitätstests mit einer Probe der Charge;
Analysieren der Probe der Charge auf das Vorhandensein von Erlotinib-Hydrochlorid Form B nach der Stabilitätsprüfung, um Erlotinib-Hydrochlorid sicherzustellen, indem ein Verfahren nach einem der Ansprüche 1 bis 4 durchgeführt wird;
Validierung der Charge für den Vertrieb nur dann, wenn die Probe der Charge nach der Stabilitätsprüfung die erforderliche polymorphe Reinheit von Erlotinib-Hydrochlorid Form A aufweist, wodurch kein Erlotinib-Hydrochlorid Form B nachweisbar ist, wenn es durch Durchführung eines Verfahrens nach einem der Ansprüche 1 bis 4 gemessen wird; und
Vertrieb der validierten Charge.

## Revendications

1. Procédé de surveillance de la pureté polymorphe d'un échantillon de chlorhydrate d'erlotinib, le procédé comprenant une analyse de diffraction sur poudre de rayons X qui comprend une résolution de différenciation et une région de scan 2-théta qui permet la détection de l'absence, ou de la présence, d'un pic 2-théta caractéristique de la forme B du chlorhydrate d'erlotinib dans la région de 6,0 à 6,4 degrés 2-théta, qui a une limite de détection qui permet la détection d'autant que, ou de moins que, 0,2 % de forme B de chlorhydrate d'erlotinib dans un échantillon test, l'analyse de diffraction sur poudre de rayons X étant effectuée à un courant de 40 mA, une tension de 40 kV, une taille d'étape de 0,02 degré 2-théta et un temps par étape de 4,0 secondes et avec une fente de divergence de 0,6 mm.

2. Procédé selon la revendication 1, qui comprend une résolution de différenciation entre pics 2-théta adjacents de pas plus d'environ 0,1 degré 2-théta.

3. Procédé selon l'une quelconque des revendications 1 à 2, qui emploie un Bruker ASX D8, avec une source de rayonnement de Cu Kα (λ = 1,54 Å) et un détecteur de type LynxEye.

4. Procédé selon l'une quelconque des revendications 1 à 3, réalisé à l'un quelconque des stades de développement de formulation : comme analyse de l'API *per se* ; comme analyse d'un mélange de formulation à granuler ; comme analyse de granulés ; et / ou comme analyse d'une forme posologique finale.

5. Procédé de préparation d'une composition pharmaceutique comprenant la forme A du chlorhydrate d'erlotinib et au moins un véhicule pharmaceutiquement acceptable, comprenant :
l'obtention d'un lot de forme A de chlorhydrate d'erlotinib ;
l'analyse du lot pour la présence de la forme B du chlorhydrate d'erlotinib de manière à assurer le chlorhydrate d'erlotinib en réalisant un processus selon l'une quelconque des revendications 1 à 4 ;
la préparation d'une composition pharmaceutique à partir du lot seulement si le lot est déterminé pour avoir la pureté polymorphe requise de forme A de chlorhydrate d'erlotinib, moyennant quoi il n'y a aucune forme B de chlorhydrate d'erlotinib détectable lorsqu'elle est mesurée en réalisant un processus selon l'une quelconque des revendications 1 à 4.

6. Procédé de préparation d'une composition pharmaceutique conditionnée comprenant la forme A du chlorhydrate d'erlotinib, comprenant :
l'obtention d'une composition pharmaceutique comprenant la forme A du chlorhydrate d'erlotinib ;
l'analyse de la composition pharmaceutique pour la présence de la forme B du chlorhydrate d'erlotinib de manière à assurer le chlorhydrate d'erlotinib en réalisant un processus selon l'une quelconque des revendications 1 à 4 ; et
le conditionnement de la composition pharmaceutique seulement si le chlorhydrate d'erlotinib est déterminé pour avoir la pureté polymorphe requise de la forme A du chlorhydrate d'erlotinib, moyennant quoi il n'y a pas de forme B détectable de chlorhydrate d'erlotinib lorsqu'elle est mesurée en réalisant un processus selon l'une quelconque des revendications 1 à 4.

7. Procédé de distribution d'un lot validé d'une composition pharmaceutique comprenant la forme A du chlorhydrate d'erlotinib et au moins un véhicule pharmaceutiquement acceptable, comprenant :
l'obtention d'un lot de la composition pharmaceutique ;
la réalisation d'un test de stabilité avec un échantillon du lot ;
l'analyse de l'échantillon du lot pour la présence de la forme B du chlorhydrate d'erlotinib après ledit test de stabilité, pour assurer le chlorhydrate d'erlotinib en réalisant un processus selon l'une quelconque des revendications 1 à 4 ;
la validation du lot pour la distribution seulement si l'échantillon du lot après le test de stabilité est déterminé pour avoir la pureté polymorphe requise de la forme A du chlorhydrate d'erlotinib, moyennant quoi il n'y a pas forme B de chlorhydrate d'erlotinib détectable lorsqu'elle est mesurée en réalisant un processus selon l'une quelconque des revendications 1 à 4 ; et
la distribution du lot validé.
